# EUROPEAN PATENT SPECIFICATION

(11) **EP 2 704 748 B1**
(45) Date of publication and mention of the grant of the patent: **15.08.2018**
(21) Application number: 12719360.5
(22) Date of filing: 02.05.2012
(51) Int. Cl.: A61K 31/4535, A61K 31/5575, A61K 9/00, A61K 47/34

(54) **BIS-(ALPHA-AMINO-DIOL-DIESTER) CONTAINING POLYESTERAMIDE FOR OPHTAMOLOGY**
BIS-(ALPHA-AMINO-DIOL-DIESTER)-HALTIGES POLYESTERAMID FÜR DIE OPHTHAMOLOGIE
POLYESTÉRAMIDE CONTENANT DU BIS-(ALPHA-AMINO-DIOL-DIESTER) POUR L'OPHTALMOLOGIE

(30) Priority: 02.05.2011 EP 11164479
(43) Date of publication of application: 12.03.2014
(73) Proprietor: DSM IP Assets B.V., 6411 TE Heerlen (NL)
(72) Inventor: FRANKEN, Astrid, NL-6100 AA Echt (NL); MIHOV, George, NL-6100 AA Echt (NL); THIES, Jens Christoph, NL-6100 AA Echt (NL)
(74) Representative: DSM Intellectual Property
(86) International application number: PCT/EP2012/058015
(87) International publication number: WO 2012/150255

(56) References cited:
- WO-A2-2007/035938
- WO-A2-2007/130477
- US-A1- 2006 009 498

## Description

The present invention relates to an intraocular polymer delivery composition comprising a polyester amide (PEA). The present invention further relates to extruded or injection molded parts sized for injection via a pharmaceutical syringe needle having a bore of 1.270 +/- 0.013 mm to 0.305 +/- 0.006 mm (18 to 30 Gauge) based on this polymer composition.

The intraocular polymer delivery composition can be used to formulate biodegradable polymer delivery compositions for time release of bioactive agent's e.g. ophthalmologic therapeutic agents in a consistent and reliable manner into the exterior or interior of the eye by biodegradation of the polymer.

The present invention is also based on the premise that PEAs can be formulated as polymer delivery compositions that may incorporate a bioactive agent into the backbone of the polymer for time release into the exterior or interior of the eye in a consistent and reliable manner by biodegradation of the polymer. The intraocular polymer delivery composition may also deliver into the exterior or interior of the eye another type of bioactive agent that is dispersed in the polymer.

Delivery of drugs intraocular is a particular problem. The eye is divided into two chambers; the anterior segment which is the front of the eye, and the posterior segment which is the back of the eye. Diseases of the anterior segment are easier to treat with formulations such as eye drops because they can be applied topically. For example, glaucoma can be treated from the front of the eye. Diseases of the retina, such as diabetic retinopathy and macular degeneration, are located in the posterior segment and are difficult to treat because drugs applied topically, such as eye drops, typically do not penetrate to the back of the eye. Drugs for these disease states have customarily been delivered by injection directly into the back of the eye. Chemists, biochemists, and chemical engineers are all looking beyond traditional polymeric and other formulations to find innovative drug transport systems. Thus, there is still a need in the art for new and better polymer delivery compositions for controlled delivery of a variety of different types of bioactive agents to target specific body sites, such as the exterior and interior tissues of the eye. In particular, there is a need in the art for new and better polymer delivery compositions for a sustained and continuous delivery of an ophthalmologic agent to the anterior or posterior segment of the eye over a longer period of time, for example in treatment of chronic diseases of the front and back of the eye where a release of ophthalmologic agents during 3-6 months would be very advantageous. It is quite important that the ophthalmologic agents are administered by controlled or sustained release technologies to attempt to increase their duration of action or reduce the toxicity of transient high general concentrations.

Polyesteramides are known in the art, in particular α-amino acid-diol-diester based polyesteramides (PEA) are known from G. Tsitlanadze, et al. J. Biomater. Sci. Polym. Edn. (2004) 15:1-24. These polyesteramides provide a variety of physical and mechanical properties as well as biodegradable profiles which can be adjusted by varying three components in the building blocks during their synthesis: naturally occurring amino acids and, therefore, hydrophobic alpha -amino acids, nontoxic fatty diols and aliphatic dicarboxylic acids.

Ophthalmic compositions comprising α-amino acid-diol-diester based polyesteramides are disclosed in WO2007/130477.

WO2007/130477 specifically refers to alpha-amino acid-diol-diester based polyesteramides (PEA) of formula IV, 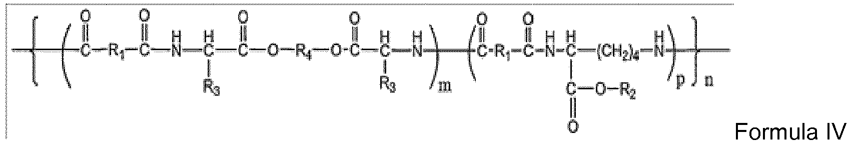 wherein:
n ranges from about 5 to about 150, m ranges about 0.1 to 0.9: p ranges from about 0.9 to 0.1; R¹ is independently selected from residues of α,ω-bis(4-carboxyphenoxy)(C₁-C₈)alkane, 3,3'-(alkanedioyldioxy)dicinnamic acid, 4,4'-(alkanedioyldioxy)dicinnamic acid, (C₂-C₂₀)alkylene, (C₂-C₂₀)alkenylene, saturated or unsaturated residues of therapeutic di-acids, residues of α,ω-alkylene dicarboxylates of formula (III), and combinations thereof; R⁵ and R⁶ in Formula (III) are each independently selected from (C₂-C₁₂)alkylene and (C₂-C₁₂)alkenylene; R² is independently selected from hydrogen, (C₁-C₁₂)alkyl, (C₂-C₈)alkyloxy, (C₂-C₂₀) alkyl, (C₆-C₁₀)aryl, and a protecting group; the R³s in individual n monomers are each independently selected from hydrogen, (C₁-C₆)alkyl, (C₂-C₆)alkenyl, (C₆-C₁₀)aryl(C₁-C₆)alkyl, and -(CH₂)₂S(CH₃); and R⁴ is independently selected from (C₂-C₂₀)alkylene, (C₂-C₂₀)alkenylene, (C₂-C₈)alkyloxy, (C₂-C₂₀)alkylene, residues of saturated or unsaturated therapeutic diols, and bicyclic-fragments of 1,4:3,6-dianhydrohexitols of formula (II), and combinations thereof. A bioactive agent may covalently bind to the carboxylic group of the lysine part or may be build into the PEA backbone a therapeutic diol or diacid.

The polyesteramides as presented by general formula IV provide a plenty of possibilities to choose R1, R2, R3, R4 and the units m, p and n. No specific selection on whatever polyesteramide is disclosed in this application, neither an example is given which shows a specific polyesteramide which provides a controlled release of ophthalmologic agents over a longer period of time.

It has been recognized that it is quite difficult to select the right monomer units and m, p, n such that a PEA can be provided which shows properties of releasing ophthalmologic agents in a consistent and reliable manner over a period of at least 3 months. It has moreover been recognized that certain polyesteramides according to Formula IV have gummy and sticky like properties which makes them impossible to be processed into injectable drug delivery devices.

The object of the present invention is therefore to provide an ocular polymer delivery composition which can processed and sized for injection via a pharmaceutical syringe needle having a bore of 1.270 +/- 0.013 mm to 0.305 +/- 0.006 mm (18 to 30) Gauge comprising an α-amino acid-diol-diester based polyesteramide from which an ophthalmologic agent can be released in a consistent and reliable manner over a period of at least 3 months.

A further object of the present invention is to provide an ophthalmic composition comprising PEA and an ophthalmologic agent which can be processed and sized for injection via a pharmaceutical syringe needle having a bore of 1.270 +/-0.013 mm to 0.305 +/- 0.006 mm (18 to 30 Gauge), and from which the release pattern is uniform and not showing a burst release in the first 24 hours.

The object of the present invention is achieved by providing an ocular polymer delivery composition sized for injection via a pharmaceutical syringe needle having a bore of 1.270 +/- 0.013 mm to 0.305 +/- 0.006 mm (18 to 30 Gauge), comprising at least one ophthalmologic agent dispersed in biodegradable poly (ester amide) (PEA) having a chemical formula described by structural formula (I), 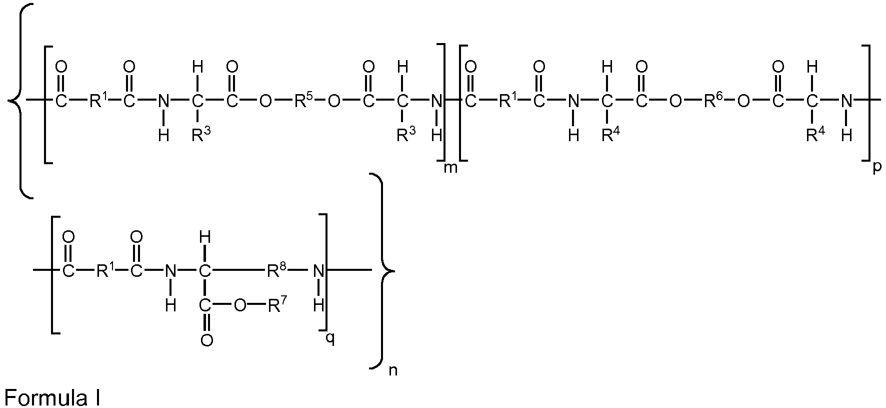 wherein
m is 0.3-0.6, p is 0.3-0.45 and q is 0.1-0.25 and wherein m+p+q=1, m, p, and
q are randomly distributed, n is about 5-100; and
   - R₁ is (CH₂)₈; (CH₂)₄;
   - R₃ and R₄ are selected from (CH₃)₂-CH-CH₂-;
   - R₅ is selected from (CH₂)₆;
   - R₆ is 1,4 :3,6-dianhydrosorbitol (DAS) according to formula II;
   - R₇ is a H, benzyl protecting group or an ophthalmologic agent; and
   - R₈ is (CH₂)₄.

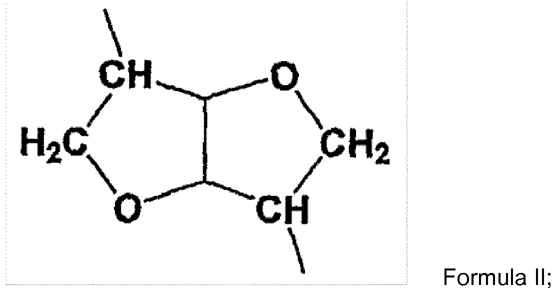

Surprisingly it has been found that these specific elected polyesteramides of formula I can be processed and sized for injection via a pharmaceutical syringe needle having a bore of 1.270 +/- 0.013 mm to 0.305 +/- 0.006 mm (18 to 30 Gauge) and provide unexpected properties in terms of release of ophthalmologic agents over a period of at least 3 months without showing a high burst release in the first 24 hours.

The PEA polymers as such are known in the art and disclosed in US2008/0299174. US2008/0299174 discloses the PEA polymers based on bis-(a-amino acid)- diol-diesters containing two bis-(a-amino acid)-based building blocks and shows the polymers to provide a significant improvement in mechanical properties. Incorporation of at least two linear saturated or unsaturated aliphatic diol residues into the two bis-(a amino acid)-based (e.g. bis-(a-amino acid)-diol-diester co-monomers of a PEA), increases the mechanical properties of the resulting polymer. Furthermore, methods are disclosed for fixing a fixation device made of the PEA's into the internal body site. The device biodegrades to create substantially biocompatible breakdown products while fixing the internal body site. Also, biocompatible surgical devices fabricated using the PEA compositions are disclosed. The disclosure is however silent about ophthalmic compositions based on the PEA's for the release of ophthalmologic agents on the longer term.

Accordingly, in a preferred embodiment, the invention provides ophthalmic compositions sized for injection via a pharmaceutical syringe needle having a bore of about 18 to 30 Gauge comprising PEA co-polymers of formula I wherein: -m is about 0.3, p is about 0.45 and q is about 0.25; m, p, and q are randomly
distributed
n is about 5-100 and wherein R₁ is (CH₂)₆, R₃ and R₄ are selected from (CH₃)₂-CH-CH₂-; R₅ is selected from (CH₂)₆; R₆ is 1,4 :3,6-dianhydrosorbitol (DAS) according to formula II; R₇ is a H, benzyl protecting group or an ophthalmologic agent and R₈ is (CH₂)₄.

In a more preferred embodiment the invention provides ophthalmic compositions sized for injection via a pharmaceutical syringe needle having a bore of 1.270 +/- 0.013 mm to 0.305 +/- 0.006 mm (18 to 30 Gauge) comprising PEA co-polymers of formula I wherein m is about 0.3, p is about 0.45, q is about 0.25, n is about 50 and R₁ is (CH₂)₈; R₃ and R₄ are selected from (CH₃)₂-CH-CH₂-; R₅ is selected from (CH₂)₆; R₆ is 1,4 :3,6-dianhydrosorbitol (DAS); R₇ is a H, benzyl protecting group or an ophthalmologic agent and R₈ is (CH₂)₄.

In an embodiment, the invention provides ophthalmic compositions sized for injection via a pharmaceutical syringe needle having a bore of 1.270 +/- 0.013 mm to 0.305 +/- 0.006 mm (18 to 30 Gauge) comprising PEA co-polymers of formula I wherein m is about 0.3, p is about 0.45, q is about 0.25, n is about 50 and R₁ is (CH₂)₄; R₃ and R₄ are selected from (CH₃)₂-CH-CH₂-; R₅ is selected from (CH₂)₆; R₆ is 1,4 :3,6-dianhydrosorbitol (DAS); R₇ is a H, benzyl protecting group or an ophthalmologic agent and R₈ is (CH₂)₄.

The PEA co-polymers preferably have an average number molecular weight (Mn) ranging from 15,000 to 200,000 Daltons. The PEA co-polymers described herein can be fabricated in a variety of molecular weights and a variety of relative proportions of the two bis-(alpha amino acid)-containing units and optional Lysine-based monomer of the co-polymer. The appropriate molecular weight for a particular use is readily determined by one of skill in the art. A suitable Mn will be in the order of about 15,000 to about 100,000 Daltons, for example from about 30,000 to about 80,000 or from about 35,000 to about 75,000. Mn is measured via GPC in THF with polystyrene as standard.

Further properties and methods of manufacturing the PEA's are disclosed in US2008/0299174.

It has been found that the nature of the PEA polymer plays an important role in defining the dimensional properties of the composition. The PEA's used in the ophthalmic composition of the present invention comprises the incorporation of a bicyclic-fragment of I,4:3,6-dianhydrohexitol as the diol residue in at least one of the two bis (a-amino acid)- based building blocks which confers a (Tg) above body temperature. By further varying the other building blocks in the PEA Tg can be adjusted. Preferably the Tg of the PEA ranges from about 40 to about 65. Tg can be measured by DSC.

Surprisingly it has been found that the release time can be tailored by varying the building blocks of the polymer and by varying the amount of the m, p, q blocks in the PEA copolymer. Moreover, the polymer/ ophthalmologic agent ratio plays an important role in the tuning of the release.

The ophthalmologic agent can be a therapeutic agent that is typically ophthalmically acceptable, such as steroidal or non-steroidal anti-inflammatory agents. Examples of steroidal anti-inflammatory agents include corticosteroids such as alclometasone dipropionate, amcinonide, amcinafel, amcinafide, beclamethasone, betamethasone, betamethasone dipropionate, betamethasone valerate, clobetasone propionate, chloroprednisone, clocortelone, Cortisol, cortisone, cortodoxone, difluorosone diacetate, descinolone, desonide, defluprednate, dihydroxycortisone, desoximetasone, dexamethasone, deflazacort, diflorasone, diflorasone diacetate, dichlorisone, esters of betamethasone, fluazacort, flucetonide, flucloronide, fludrotisone, fluorocortisone, flumethasone, flunisolide, fluocinonide, fluocinolone, fluocinolone acetonide, flucortolone, fluperolone, fluprednisolone, fluroandrenolone acetonide, fluocinolone acetonide, flurandrenolide, fluorametholone, fluticasone propionate, hydrocortisone, hydrocortisone butyrate, hydrocortisone valerate, hydrocortamate, loteprendol, medrysone, meprednisone, methylprednisone, methylprednisolone, mometasone furoate, paramethasone, paramethasone acetate, prednisone, prednisolone, prednidone, triamcinolone acetonide, triamcinolone hexacatonide, and triamcinolone, salts thereof, derivatives thereof, and mixtures thereof. As used herein, the term "derivative" refers to any substance which is sufficiently structurally similar to the material which it is identified as a derivative so as to have substantially similar functionality or activity, for example, therapeutic effectiveness, as the material when the substance is used in place of the material. Other steroids which may be useful in the present compositions include, without limitation, glucocorticoids, androgenic steroids, estrogenic steroids, and non-estrogenic steroids.

The ophthalmologic agent may be present in an amount in the range of about 1 percent or less to about 5 percent, about 10 percent, about 20 percent, about 25 percent or about 30 percent or more (w/v) of the composition. The amount is dependent on the nature, the therapeutic dosage and the potency of a particular ophthalmologic agent.

The present ophthalmic compositions may comprise other therapeutic agents instead of or in addition to the anti -inflammatory agents disclosed herein. For example, therapeutic agents may include without limitation retinoids, prostaglandins, tyrosine kinase inhibitors, adrenoreceptor agonists or antagonists, dopaminergic agonists, cholinergic agonists, carbonic anhydrase inhibitors, guanylate cyclase activators, cannabinoids, endothelin, adenosine agonists, antianagiogenic compounds, angiostatic compounds, neuroprotectants, and the like and mixtures thereof. The therapeutic component may also include, analgesics, or antipyretics; antihistamines, antibiotics, beta blockers, anti-neoplastic agents, immunosupressive agents, antiviral agents, antioxidants and the like and mixtures thereof.

Non-limiting examples of non-steroidal anti-inflammants, analgesics, and antipyretics, include aspirin, acetaminophen, ibuprofen, naproxen, diclofenac, etodolac, fenoprofen, indomethacin, ketoprofen, oxaprozin, piroxicam, sulindac, diflunisal, mefenamic acid, derivatives thereof, and the like and mixtures thereof.

Examples of antihistamines include, and are not limited to ketotifen, loradatine, hydroxyzine, diphenhydramine, chlorpheniramine, brompheniramine, cyproheptadine, terfenadine, clemastine, triprolidine, carbinoxamine, diphenylpyraline, phenindamine, azatadine, tripelennamine, dexchlorpheniramine, dexbrompheniramine, methdilazine, and trimprazine doxylamine, pheniramine, pyrilamine, chiorcyclizine, thonzylamine, derivatives thereof, and the like and mixtures thereof. Examples of antibiotics include without limitation, cefazolin, cephradine, cefaclor, cephapirin, ceftizoxime, cefoperazone, cefotetan, cefutoxime, cefotaxime, cefadroxil, ceftazidime, cephalexin, cephalothin,, cefamandole, cefoxitin, cefonicid, ceforanide, ceftriaxone, cefadroxil, cephradine, cefuroxime, ampicillin, amoxicillin, cyclacillin, ampicillin, penicillin G, penicillin V potassium, piperacillin, oxacillin, bacampicillin, cloxacillin, ticarcillin, azlocillin, carbenicillin, methicillin, nafcillin, erythromycin, tetracycline, doxycycline, minocycline, aztreonam, chloramphenicol, ciprofloxacin hydrochloride, clindamycin, metronidazole, gentamicin, lincomycin, tobramycin, vancomycin, polymyxin B sulfate, colistimethate, colistin, azithromycin, augmentin, sulfamethoxazole, trimethoprim, derivatives thereof, and the like and mixtures thereof.

Examples of beta blockers include without limitation acebutolol, atenolol, labetalol, metoprolol, propranolol, derivatives thereof, and the like and mixtures thereof. Examples of antineoplastic agents include without limitation adriamycin, cyclophosphamide, actinomycin, bleomycin, duanorubicin, doxorubicin, epirubicin, mitomycin, methotrexate, fluorouracil, carboplatin, carmustine (BCNU), methyl-CCNU, cisplatin, etoposide, interferons, camptothecin and derivatives thereof, phenesterine, taxol and derivatives thereof, taxotere and derivatives thereof, vinblastine, vincristine, tamoxifen, etoposide, piposulfan, cyclophosphamide, and flutamide, derivatives thereof, and the like and mixtures thereof.

Examples of immunosuppressive agents include without limitation cyclosporine, azathioprine, tacrolimus, derivatives thereof, and the like and mixtures thereof. Examples of antiviral agents include without limitation interferon gamma, zidovudine, amantadine hydrochloride, ribavirin, acyclovir, valciclovir, dideoxycytidine, derivatives thereof, and the like and mixtures thereof.

Examples of antioxidant agents include without limitation ascorbate, alpha-tocopherol, mannitol, reduced glutathione, various carotenoids, cysteine, uric acid, taurine, tyrosine, superoxide dismutase, lutein, zeaxanthin, cryotpxanthin, astazanthin, lycopene, N-acetyl-cysteine, carnosine, gamma- glutamylcysteine, quercitin, lactoferrin, dihydrolipoic acid, citrate, Ginkgo Biloba extract, tea catechins, bilberry extract, vitamins E or esters of vitamin E, retinyl palmitate, derivatives thereof, and the like and mixtures thereof. Other therapeutic agents include without limitation squalamine, carbonic anhydrase inhibitors, alpha agonists, prostamides, prostaglandins, antiparasitics, antifungals, derivatives thereof, and the like and mixtures thereof. Examples of prostaglandins are latanoprost, bimatoprost, tafluprost, travoprost. Further examples include aminosterols other than squalamine that have antiangiogenic activity. Another therapeutic agent may be anecortave acetate, or similar agents or compounds which have anti-angiogenic properties without substantial undesirable effects.

The therapeutic agent of the present compositions may include any and all salts, and prodrugs or precursors of the therapeutic agents, including those specifically identified herein.

The ophthalmic composition according to the present invention may further a second or even third ophthalmic agent.

In a further embodiment, the ophthalmic composition according to the present invention may be formed of the PEA polymer as such or of a blend with one or more other polymers. Representative polymers include, but are not limited to, poly(ester amide), polyhydroxyalkanoates (PHA), poly(3-hydroxyalkanoates) such as poly(3- hydroxypropanoate), poly(3-hydroxybutyrate), poly(3-hydroxyvalerate), poly(3-hydroxyhexanoate), poly(3-hydroxyheptanoate) and poly(3-hydroxyoctanoate), poly(4-hydroxyalkanaote) such as poly(4-hydroxybutyrate), poly(4-hydroxyvalerate), poly(4-hydroxyhexanote), poly(4-hydroxyheptanoate), poly(4-hydroxyoctanoate) and copolymers including any of the 3-hydroxyalkanoate or 4-hydroxyalkanoate monomers described herein or blends thereof, poly(D,L-lactide), poly(L-lactide), polyglycolide, poly(D,L-lactide-co- glycolide), poly(L-lactide-co-glycolide), polycaprolactone, poly(lactide-co-caprolactone), poly(glycolide-co-caprolactone), poly(dioxanone), poly(ortho esters), poly(trimethylene carbonate), polyphosphazenes, poly(anhydrides), poly(tyrosine carbonates) and derivatives thereof, poly(tyrosine ester) and derivatives thereof, poly(imino carbonates), poly(glycolic acid-co-trimethylene carbonate), polyphosphoester, polyphosphoester urethane, poly(amino acids), poly(ethylene glycol) (PEG), copoly(ether-esters) (e.g. PEO/PLA), polyalkylene oxides such as poly(ethylene oxide), poly(propylene oxide), poly(ether ester), polyalkylene oxalates, poly(aspirin), biomolecules such as collagen, chitosan, alginate, fibrin, fibrinogen, cellulose, starch, collagen, dextran, dextrin, fragments and derivatives of hyaluronic acid, heparin, fragments and derivatives of heparin, glycosamino glycan (GAG), GAG derivatives, polysaccharide, elastin, chitosan, alginate, or combinations thereof.

The present invention further relates to an extruded or injection molded parts comprising the ophthalmic composition according to the present invention. The extruded or injection molded parts are sized for injection via a pharmaceutical syringe needle having a bore of 1.270 +/-0.013 mm to 0.305 +/- 0.006 mm (18 to 30 Gauge) and can be used to treat, prevent, or ameliorate a medical ophthalmic condition.

As used herein, the extruded or injection molded parts may be any suitable medical substrate that can be injected in a human or veterinary patient via a pharmaceutical syringe needle having a bore of 1.270 +/- 0.013 mm to 0.305 +/- 0.006 mm (18-30 Gauge), preferably 0.914 +/- 0.006 mm to 0.305 +/- 0.006 mm (20-30 Gauge), more preferably a bore of 0.711 +/-0.006 mm to 0.013 +/- 0.006 mm (22-29 Gauge), most preferably a bore of 0.559 +/- 0.006 mm to 0.305 +/- 0.006 mm (24-28 Gauge). Examples of such medical substrates include particles, fibers, tubes or rods.

The medical substrates may be fabricated via extrusion of via injection molding or may be based on different layers of various polymers of the present invention where m, p and n vary so as to achieve a given mechanical property e.g. lubricity for ease of injectability and drug release behavior.

The present invention further relates to injectable fluids that gel or solidify upon contact with physiological fluid or at body temperature comprising the composition of the present invention.

The present disclosure further relates to a method of delivering at least one ophthalmologic agent to the interior or exterior of the eye of a subject, said method comprising: administering the ophthalmic composition of the present invention or an injectable fluid into the interior or exterior of the eye of the subject for sustained and controlled release of the ophthalmologic agent. In at least one embodiment, the composition can be administered without accessing the subretinal space of the eye. For example, a method of treating a patient may include injecting the composition directly into the posterior chamber of the eye. In other embodiments, a method of treating a patient may comprise administering the composition to the patient by at least one of intravitreal injection, subconjuctival injection, sub-tenon injections, retrobulbar injection, and suprachoroidal injection.

Among the diseases/conditions which can be treated or addressed in accordance with the present disclosure include, without limitation, the following: Maculopathies/retinal degeneration such as non-Exudative Age Related Macular Degeneration (ARMD), Exudative Age Related Macular Degeneration (ARMD), wet macular degeneration.

Neovascularization, Diabetic Retinopathy, Acute Macular Neuroretinopathy, Central Serous Chorioretinopathy, Cystoid Macular Edema, Diabetic Macular Edema.

Uveitis/Retinitis/Choroiditis: Acute Multifocal Placoid Pigment Epitheliopathy, Behcet's Disease, Birdshot Retinochoroidopathy,

Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpignous Choroiditis, Subretinal Fibrosis and Uveitis Syndrome, Vogt-Koyanagi-Harada Syndrome.

### Vascular disease/Exudative diseasas: Retinal Arterial

Occlusive Disease, Central Retinal Vein Occlusion, Disseminated Intravascular Coagulopathy, Branch Retinal Vein Occlusion, Hypertensive Fundus Changes, Ocular Ischemic Syndrome, Retinal Arterial Microaneurysms, Coat's Disease, Parafoveal Telangiectasis, Hemi-Retinal Vein Occlusion, Papillophlebitis, Central Retinal Artery Occlusion, Branch Retinal Artery Occlusion, Carotid Artery Disease (CAD), Frosted Branch Angitis, Sickle Cell Retinopathy and other Hemoglobinopathies, Angioid Streaks, Familial Exudative Vitreoretinopathy, Eales Disease.
Traumatic/surgical: Sympathetic Ophthalmia, Uveitic Retinal Disease, Retinal Detachment, Trauma, Laser, PDT, Photocoagulation,
Hypoperfusion during surgery, Radiation Retinopathy, Bone Marrow Transplant Retinopathy.
Proliferative disorders: Proliferative Vitreal Retinopathy and Epiretinal Membranes, Proliferative Diabetic Retinopathy.
Infectious disorders: Ocular Histoplasmosis, Ocular Toxocariasis, Presumed Ocular Histoplasmosis Syndrome (POHS),
Endophthalmitis, Toxoplasmosis, Retinal Diseases Associated with HIV Infection, Choroidal Disease Associated with HIV Infection, Uveitic Disease Associated with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis.
Genetic disorders: Retinitis Pigmentosa, Systemic Disorders with Accosiated Retinal Dystrophies, Congenital Stationary Night Blindness, Cone Dystrophies, Stargardt's Disease and Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum.
Retinal tears/holes: Retinal Detachment, Macular Hole, Giant Retinal Tear.
Tumors: Retinal Disease Associated with Tumors, Congenital Hypertrophy of the RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, Intraocular Lymphoid Tumors.
Miscellaneous: Punctate Inner Choroidopathy, Acute Posterior Multifocal Placoid Pigment Epitheliopathy, Myopic Retinal Degeneration, Acute Retinal Pigment Epithelitis and the like.

The invention will now be further and specifically described by the following examples.

### MATERIALS

PEA-III-Ac Bz polymers are used in the following examples. A more extended description of PEA-III-Ac Bz is poly-8-[(L-Leu-DAS)_{0.45}(L-Leu-6)_{0.3}-[L-Lys(Bz)]_{0.25}. Structure is given in Formula III. The fractions indicate overall fractions of the monomers in the synthesis.

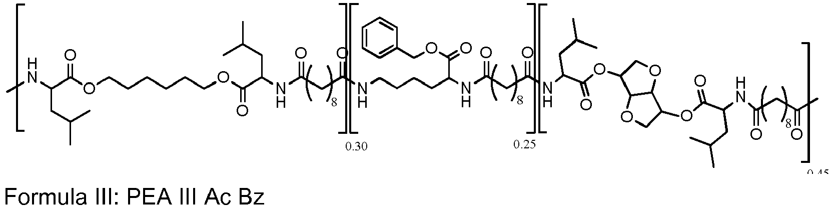

### Synthesis of PEA III Ac Bz

Trietylamine (30.9 mL, 0.222 mole, 2.2 eq) and N,N-dimethylformamide (53.07 mL, 0.689 mole) were added to a mixture of Di-OSu-sebacinate (39.940 g, 0.1008 mole, 1.0 eq), L-leucine(6)-2TosOH (20.823 g, 0.0302 mole, 0.30 eq), L-leucine-(DAS)-2TosOH (32.503 g, 0.0453 mole, 0.45 eq) and L-lysine(Bz)-2TosOH (14.628 g, 0.0252 mole, 0.25 eq) in a nitrogen flushed 500mL round bottomed flask equipped with a overhead stirrer at room temperature. The subsequent mixture was heated to 60°C to allow the reaction to proceed and monitored by GPC analysis in THF. After 36 hours a stable molecular weight was obtained, subsequently a portion of L-leucine(6)-2TosOH (4.338 g, 0.0063 mole) along with triethylamine (1.76 mL, 0.0126 mole) and N,N-dimethylformamide (4.54 mL, 0.0590 mole) was added to terminate the polymerization reaction. The mixture was heated additionally for 24 hours after which the viscous solution was further diluted with N,N-dimethylformamide (407.85 g, 5.301 mole) and allowed to cool to room temperature. At room temperature acetic anhydride (1.89 mL, 0.0199 mole) was added to acylate the amino functional end groups of the polymer. The mixture was stirred at room temperature for 24 hours. In scheme 1 the general reaction is shown. 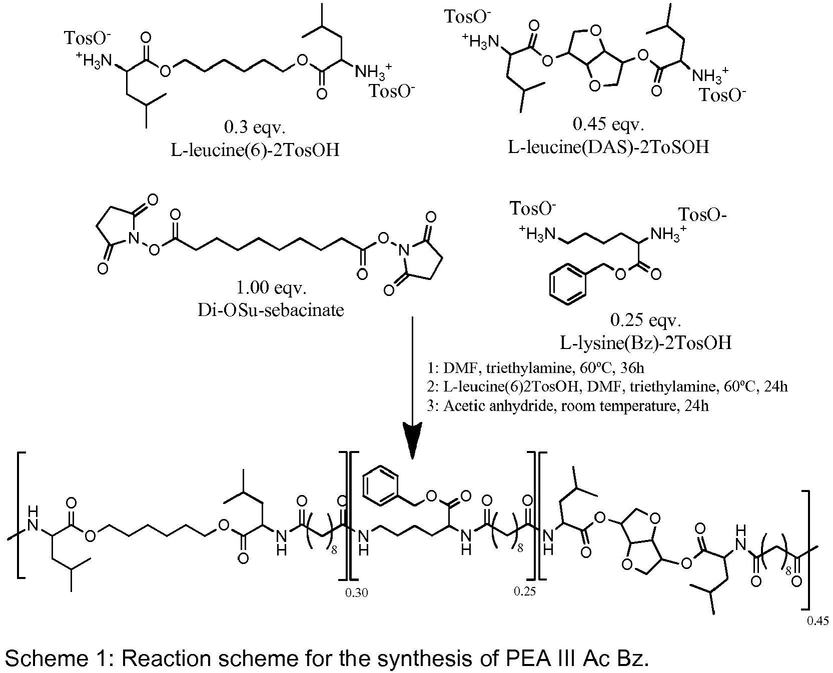 The obtained crude polymer mixture was precipitated in water in a 10:1 ratio (water: reaction mixture). The polymer was collected and dissolved in ethanol (500 mL, 8.57 mole) and the procedure was repeated a second time. The polymer was again dissolved in ethanol (500 mL, 8.57 mole) and precipitated in ethylacetate (5000 mL, 50.91 mole) by drop wise addition to a stirring solution. The precipitated polymer was washed with two portions ethylacetate (100 mL, 1.00 mole), dried and dissolved in ethanol (500 mL, 8.57 mole) and filtered over a 0.2 µm PTFE membrane filter. The filtered polymer solution was dried under reduced pressure at 65 °C.
Yield 75%, Mn =50 kDa (Gel Permeation Chromatography (GPC) in THF relative to polystyrene standards. Glass transition temperatures were determined by Differential Scanning Calorimetry (DSC). Measurements were taken from second heating, with a heating rate of 10 °C/min., Tg= 48 °C.

Films were prepared from these polymers.

Latanoprost was purchased from Cayman Chemicals (Item # 16812, Batch 188844-14.

Ketotifen fumarate was purchased from Sifavator.

Other materials and chemicals used in this feasibility phase are generally obtained from Sigma-Aldrich and VWR/Merck. Phosphate buffered saline (PBS) was obtained from BioChrom. Tetrahydrofuran (THF) was obtained from Prolabo. Acetonitril (ACN), ethanol and phosphoric acid (H₃PO₄) were obtained from Merck. MilliQ was processed internally. Deuterated THF was obtained from Sigma Aldrich.

### METHODS

For film casting, solutions were prepared in a suitable solvent resulting in solutions with viscosities low enough for solvent casting. Exact amounts of PEA-III ac Bz and API (Active Pharmaceutical Ingredient) were weighed and dissolved with a suitable solvent. The API payload is determined over the polymer weight. API payload of 10% was used. The API/polymer/solvent formulations were poured into petri dishes (4 cm diameter) and covered to reduce evaporation rates of the solvent and prevent formation of air bubbles. The solvent was evaporated for 2 days at rT in a fumehood followed by 2 days @ 750 mBar, 1 day @ 500 mBar and 2 days at full vacuum. From the resulting films, discs were cut with diameter of 10mm and approximate thicknesses of 500 µm.

### 1H NMR Measurements

A sample was prepared by dissolving -10 mg of material in 1 ml of deuterated THF in a 10 ml glass vial. The sample was allowed sufficient time for complete dissolution. ¹H NMR spectra was recorded on a 300 MHz Bruker Avance 300, ARX 400 NMR instrument.

### HPLC quantification method

For the quantitative analysis of the release of the Latanoprost sample a Waters e2695 Alliance HPLC with a photodiode array detector was used. An isocratic HPLC method was used with an Agilent Zorbax Eclipse XBD-C18 4.6 x 250 mm, 5 µm column. The mobile phase was Acetonitrile / H₂O (60 / 40 containing 0.05% TFA) and the flow was 1.0 ml/min. Column temperature was set to 25 °C and sample temperature to 15 °C. Samples were measured at a wavelength of 210 nm.

Release studies were performed in phosphate buffered saline (PBS). An accurate volume of buffer was measured into a glass vial containing the sample, with a volume depending on the

### EXAMPLE I

This example describes Latanoprost stability in PEA III formulations and the release of latanoprost from PEA-III Ac Bz discs.

Stability of Latanoprost in formulations and compatibility with film casting procedure was evaluated by ¹H NMR. Analysis of spectra of initial PEA III formulations with Latanoprost as well as formulations stored @ 4 °C for 3 months did not indicate any degradation of API.

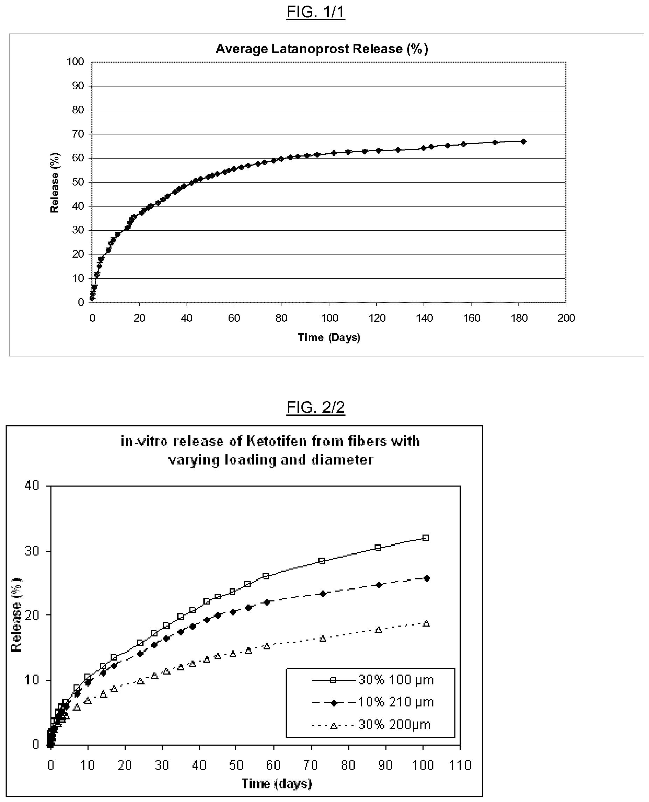

Time-exchange release studies were conducted on representative discs with 10% Latanoprost. The weight per disc was around 20mg and was recorded accurately. A volume of 5 mL of PBS was used for each release article. Full volume of PBS was fully exchanged at each time point. The amount of Latanoprost was measured using HPLC and the release curve in total % API is presented in Figure 1.

### EXAMPLE II

Fiber extrusions were performed using a 5 cc twin-screw mini-extruder.

Ketotifen fumarate and PEA-III-Ac Bz were co-dissolved in ethanol, then film-casted and allowed to dry. Films were cut in pieces and then cryo-milled. The resulting flakes were brought into the hopper at the top of the heated barrel via a steel funnel. A temperature gradient was applied to the barrel of 100 °C to 130 °C and 140 °C from top to middle to bottom part. In-vitro release studies were performed in phosphate buffered saline (PBS). A volume of 200 ul of buffer was pipetted into a glass vial containing the sample, with a volume depending on the expected API release. The buffer was removed and replaced with fresh buffer at defined time points, typically after 1 hour, 4 hours, 1 day, 2 days, 4 days, 7 day etc. until completion of the release study. During the release study the samples were kept in glass vials at 37 °C while shaking. The PBS solution with released API was transferred to HPLC vials and stored refrigerated until HPLC analysis. The release study was continued until no API was detectable anymore by HPLC or until 100% of the loaded API had been released. All release experiments were carried out with n=3 or n=4. Sink conditions were applied to avoid influence of saturation of the PBS on release kinetics. The amount of ketotifen fumarate was measured using HPLC and the release curves in total % API is presented in Figure 2.

## Claims

1. An ocular polymer delivery composition sized for injection via a pharmaceutical syringe needle having a bore of 1.270 +/- 0.013 mm to 0.305 +/- 0.006 mm (18 to 30 Gauge), comprising at least one ophthalmologic agent dispersed in biodegradable poly (ester amide) (PEA) having a chemical formula described by structural formula (I), 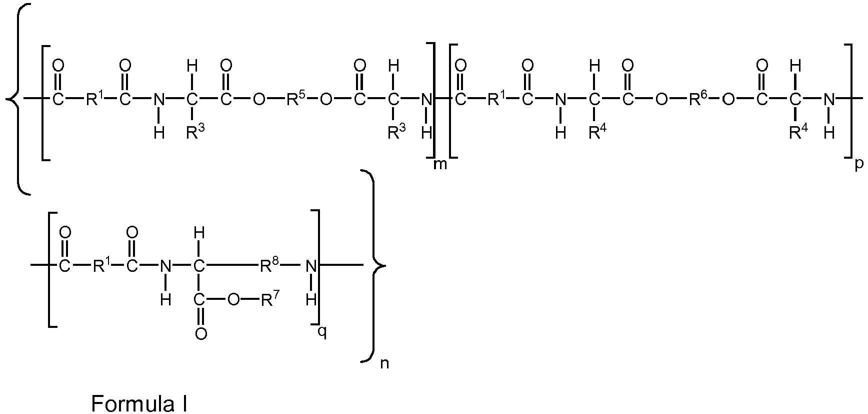 wherein
m is 0.3-0.6, p is 0.3-0.45 and q is 0.1-0.25 and wherein m+p+q=1, m,p,q are randomly distributed within formula I, n is 5-100; and
- R₁ is (CH₂)₈ or (CH₂)₄;
- R₃ and R₄ are selected from (CH₃)₂-CH-CH₂-;
- R₅ is selected from (CH₂)₆;
- R₆ is 1,4 :3,6-dianhydrosorbitol (DAS) according to formula II;
- R₇ is a H, benzyl protecting group or an ophthalmologic agent; and
- R₈ is (CH₂)₄.

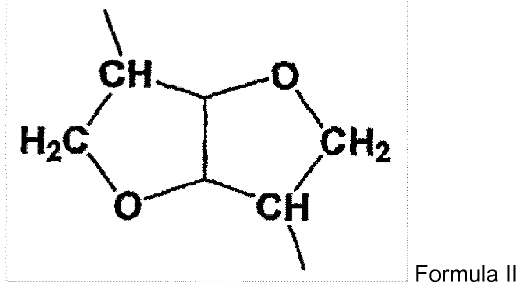

2. An ocular polymer delivery composition sized for injection via a pharmaceutical syringe needle having a bore of 1.270 +/- 0.013 mm to 0.305 +/- 0.006 mm (18 to 30 Gauge), comprising at least one ophthalmologic agent dispersed in biodegradable poly (ester amide) (PEA) having a chemical formula described by structural formula (I), 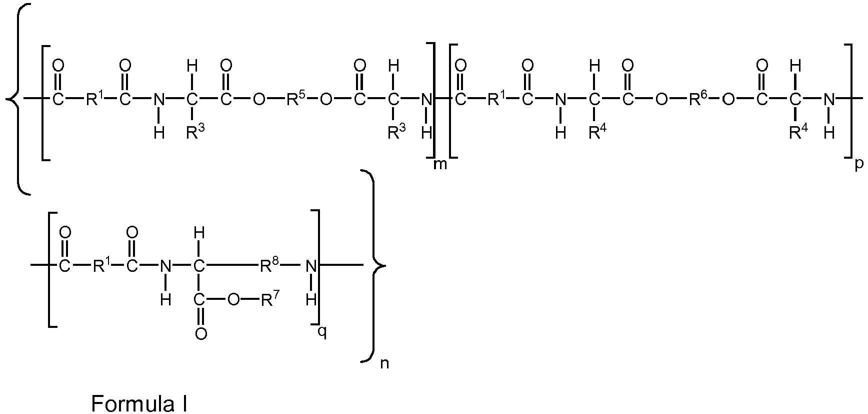 wherein
- m is 0.3, p is 0.45 and q is 0.25;
- the individual building blocks of ratio m,p and q are randomly distributed along the polymer chain within formula I, n is 5-100; and
- R₁ is (CH₂)₆;
- R₃ and R₄ are selected from (CH₃)₂-CH-CH₂-;
- R₅ is selected from (CH₂)₆;
- R₆ is 1,4 :3,6-dianhydrosorbitol (DAS) according to formula II;
- R₇ is a H, benzyl protecting group or an ophthalmologic agent; and
- R₈ is (CH₂)₄.

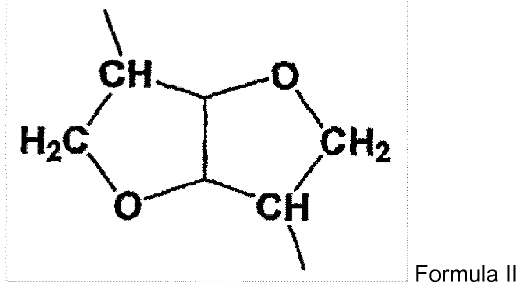

3. An ocular polymer delivery composition according to claim 1 wherein
- m is 0.6, p is 0.3 and q is 0.1;
- the individual building blocks of ratio m, p and q are randomly distributed along the polymer chain within formula I, n is 5-100; and
- R₁ is (CH₂)₄;
- R₃ and R₄ are selected from (CH₃)₂-CH-CH₂-;
- R₅ is selected from (CH₂)₆;
- R₆ is 1,4 :3,6-dianhydrosorbitol (DAS) according to formula II;
- R₇ is a H, benzyl protecting group or an ophthalmologic agent; and
- R₈ is (CH₂)₄.

4. An ocular polymer delivery composition according to any one of the claims 1-3 wherein composition is sized for injection via a pharmaceutical syringe needle having a bore of 0.559 +/- 0.006 mm to 0.305 +/- 0.006 mm (24-28 Gauge).

5. Particles, fibers, tubes, rods comprising the ocular polymer composition according to any one of the claims 1-4.

6. Injectable fluid that gels or solidifies upon contact with physiological fluid or at body temperature comprising the composition according to any one of the claims 1-4.

7. An ocular polymer delivery composition according to claim 1 wherein
- m is 0.6, p is 0.3 and q is 0.1;
- the individual building blocks of ratio m, p and q are randomly distributed along the polymer chain within formula I, n is 5-100; and
- R₁ is (CH₂)₈;
- R₃ and R₄ are selected from (CH₃)₂-CH-CH₂-;
- R₅ is selected from (CH₂)₆;
- R₆ is 1,4 :3,6-dianhydrosorbitol (DAS) according to formula II;
- R₇ is a H, benzyl protecting group or an ophthalmologic agent; and
- R₈ is (CH₂)₄.

## Patentansprüche

1. Okulare Polymerabgabezusammensetzung bemessen zur Injektion über eine pharmazeutische Spritzennadel mit einem Bohrloch von 1,270 +/- 0,013 mm bis 0,305 +/- 0,006 mm (18 bis 30 Gauge),
umfassend wenigstens ein ophthalmologisches Mittel dispergiert in biologisch abbaubarem Poly(esteramid) (PEA) mit einer chemischen Formel beschrieben durch Strukturformel (I), 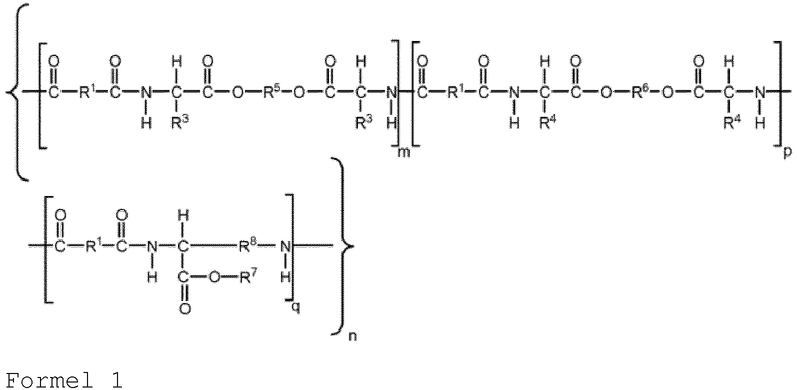 worin
m 0,3 - 0,6 ist, p 0,3 - 0,45 ist und q 0,1 - 0,25 ist und worin m + p + q = 1, m, p, q zufällig in Formel I verteilt sind, n 5 - 100 ist; und
- R₁ (CH₂)₈ oder (CH₂)₄ ist;
- R₃ und R₄ aus (CH₃)₂-CH-CH₂- ausgewählt sind;
- R₅ aus (CH₂)₆ ausgewählt ist;
- R₆ 1,4:3,6-Dianhydrosorbitol (DAS) gemäß Formel II ist;
- R₇ H, eine Benzylschutzgruppe oder ein ophthalmologisches Mittel ist; und
- R₈ (CH₂)₄ ist.

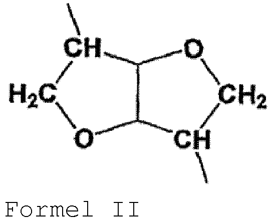

2. Okulare Polymerabgabezusammensetzung bemessen zur Injektion über eine pharmazeutische Spritzennadel mit einem Bohrloch von 1,270 +/- 0,013 mm bis 0,305 +/- 0,006 mm (18 bis 30 Gauge),
umfassend wenigstens ein ophthalmologisches Mittel dispergiert in biologisch abbaubarem Poly(esteramid) (PEA) mit einer chemischen Formel beschrieben durch Strukturformel (I), 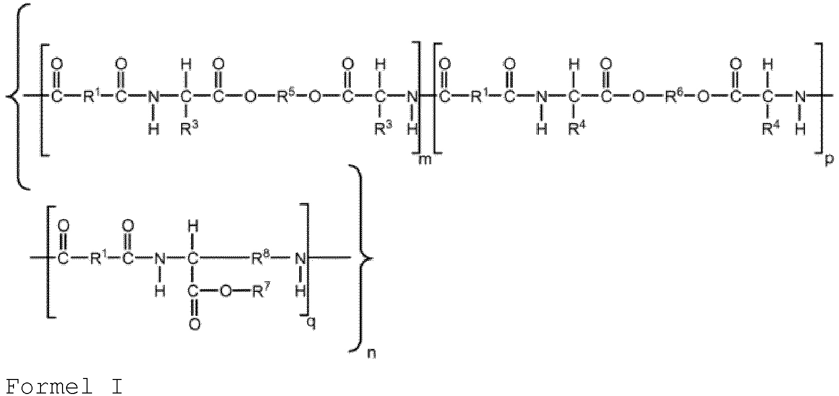 worin
- m 0,3 ist, p 0,45 ist und q 0,25 ist;
- die einzelnen Bausteine des Verhältnisses m, p und q zufällig entlang der Polymerkette in Formel I verteilt sind, n 5 - 100 ist; und
- R₁ (CH₂)₆ ist;
- R₃ und R₄ aus (CH₃)₂-CH-CH₂- ausgewählt sind;
- R₅ aus (CH₂)₆ ausgewählt ist;
- R₆ 1,4:3,6-Dianhydrosorbitol (DAS) gemäß Formel II ist;
- R₇ H, eine Benzylschutzgruppe oder ein ophthalmologisches Mittel ist; und
- R₈ (CH₂)₄ ist.

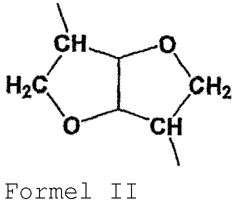

3. Okulare Polymerabgabezusammensetzung nach Anspruch 1, wobei
- m 0,6 ist, p 0,3 ist und q 0,1 ist;
- die einzelnen Bausteine vom Verhältnis m, p und q zufällig entlang der Polymerkette in Formel I verteilt sind, n 5 - 100 ist; und
- R₁ (CH₂)₄ ist;
- R₃ und R₄ aus (CH₃)₂-CH-CH₂- ausgewählt sind;
- R₅ aus (CH₂)₆ ausgewählt ist;
- R₆ 1,4:3,6-Dianhydrosorbitol (DAS) gemäß Formel II ist;
- R₇ H, eine Benzylschutzgruppe oder ein ophthalmologisches Mittel ist; und
- R₈ (CH₂)₄ ist.

4. Okulare Polymerabgabezusammensetzung nach einem der Ansprüche 1 -3, wobei die Zusammensetzung zur Injektion über eine pharmazeutische Spritzennadel mit einem Bohrloch von 0,559 +/- 0,006 mm bis 0,305 +/- 0,006 mm (24 bis 28 Gauge) bemessen ist.

5. Partikel, Fasern, Röhrchen, Stäbchen, umfassend die okulare Polymerzusammensetzung nach einem der Ansprüche 1 - 4.

6. Injizierbare Flüssigkeit, die bei Kontakt mit physiologischer Flüssigkeit oder bei Körpertemperatur geliert oder sich verfestigt, umfassend die Zusammensetzung nach einem der Ansprüche 1 - 4.

7. Okulare Polymerabgabezusammensetzung nach Anspruch 1, worin
- m 0,6 ist, p 0,3 ist und q 0,1 ist;
- die einzelnen Bausteine vom Verhältnis m, p und q zufällig entlang der Polymerkette in Formel I verteilt sind, n 5 - 100 ist; und
- R₁ (CH₂)₈ ist;
- R₃ und R₄ aus (CH₃)₂-CH-CH₂- ausgewählt sind;
- R₅ aus (CH₂)₆ ausgewählt ist;
- R₆ 1,4:3,6-Dianhydrosorbitol (DAS) gemäß Formel II ist;
- R₇ H, eine Benzylschutzgruppe oder ein ophthalmologisches Mittel ist; und
- R₈ (CH₂)₄ ist.

## Revendications

1. Composition d''administration de polymère oculaire dimensionnée pour injection via une aiguille de seringue pharmaceutique ayant un calibre de 1,270 +/- 0,013 mm à 0,305 +/- 0,006 mm (18 à 30 G),
comprenant au moins un agent ophtalmologique dispersé dans un poly(esteramide) (PEA) biodégradable ayant une formule chimique décrite par la formule structurale (I), 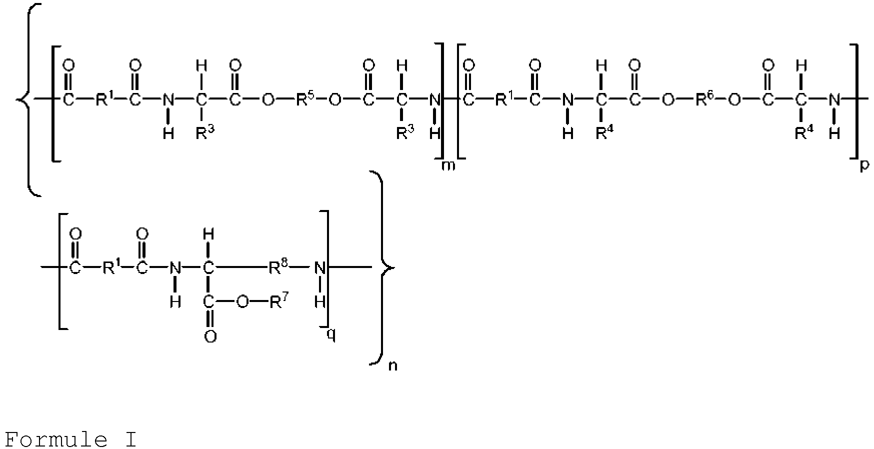 dans laquelle
m est 0,3 à 0,6, p est 0,3 à 0,45 et q est 0,1 à 0,25 et dans laquelle m+p+q=1, m, p, q sont distribués de façon aléatoire dans la formule I, n est 5 à 100 ; et
- R₁ est (CH₂)₈ ou (CH₂)₄ ;
- R₃ et R₄ sont choisis parmi (CH₃)₂-CH-CH₂- ;
- R₅ est choisi parmi (CH₂)₆ ;
- R₆ est le 1,4:3,6-dianhydrosorbitol (DAS) selon la formule II ;
- R₇ est H, un groupe protecteur benzylique ou un agent ophtalmologique ; et
- R₈ est (CH₂)₄.

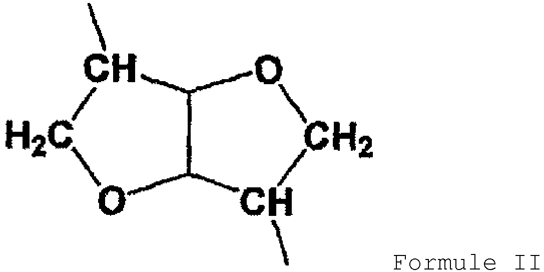

2. Composition d'administration de polymère oculaire dimensionnée pour injection via une aiguille de seringue pharmaceutique ayant un calibre de 1,270 +/- 0,013 mm à 0,305 +/- 0,006 mm (18 à 30 G), comprenant au moins un agent ophtalmologique dispersé dans un poly(esteramide) (PEA) biodégradable ayant une formule chimique décrite par la formule structurale (I), 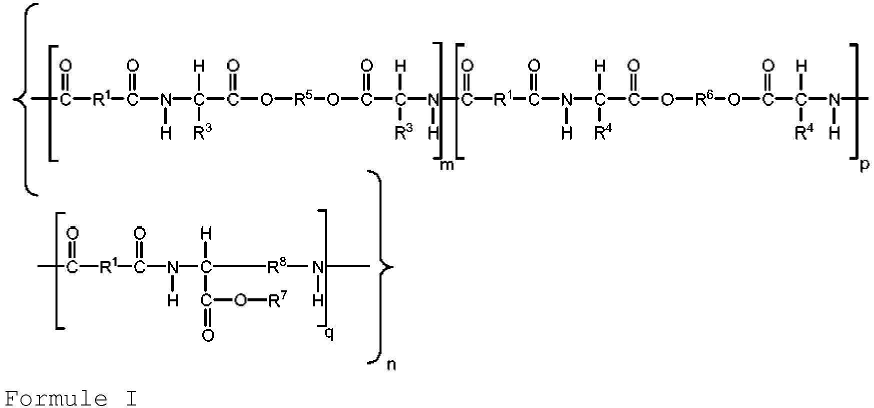 dans laquelle
- m est 0,3, p est 0,45 et q est 0,25 ;
- les synthons individuels de rapport m, p et q sont distribués de façon aléatoire le long de la chaîne de polymère dans la formule I, n est 5 à 100 ; et
- R₁ est (CH₂)₆ ;
- R₃ et R₄ sont choisis parmi (CH₃)₂-CH-CH₂- ;
- R₅ est choisi parmi (CH₂)₆ ;
- R₆ est le 1,4:3,6-dianhydrosorbitol (DAS) selon la formule II ;
- R₇ est H, un groupe protecteur benzylique ou un agent ophtalmologique ; et
- R₈ est (CH₂)₄.

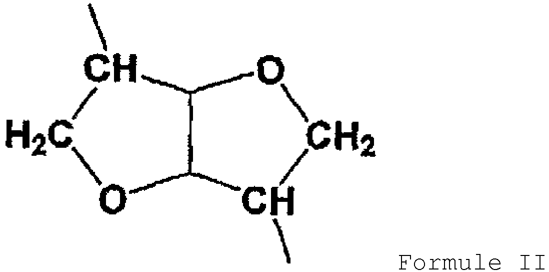

3. Composition d'administration de polymère oculaire selon la revendication 1 dans laquelle
- m est 0,6, p est 0,3 et q est 0,1 ;
- les synthons individuels de rapport m, p et q sont distribués de façon aléatoire le long de la chaîne de polymère dans la formule I, n est 5 à 100 ; et
- R₁ est (CH₂)₄ ;
- R₃ et R₄ sont choisis parmi (CH₃)₂-CH-CH₂- ;
- R₅ est choisi parmi (CH₂)₆ ;
- R₆ est le 1,4:3,6-dianhydrosorbitol (DAS) selon la formule II ;
- R₇ est H, un groupe protecteur benzylique ou un agent ophtalmologique ; et
- R₈ est (CH₂)₄.

4. Composition d'administration de polymère oculaire selon l'une quelconque des revendications 1 à 3, la composition étant dimensionnée pour injection via une aiguille de seringue pharmaceutique ayant un calibre de 0,559 +/- 0,006 mm à 0,305 +/- 0,006 mm (24-28 G).

5. Particules, fibres, tubes, tiges comprenant la composition de polymère oculaire selon l'une quelconque des revendications 1 à 4.

6. Fluide injectable qui gélifie ou solidifie au contact d'un fluide physiologique ou à température corporelle comprenant la composition selon l'une quelconque des revendications 1 à 4.

7. Composition d'administration de polymère oculaire selon la revendication 1 dans laquelle
- m est 0,6, p est 0,3 et q est 0,1 ;
- les synthons individuels de rapport m, p et q sont distribués de façon aléatoire le long de la chaîne de polymère dans la formule I, n est 5 à 100 ; et
- R₁ est (CH₂)₈ ;
- R₃ et R₄ sont choisis parmi (CH₃)₂-CH-CH₂- ;
- R₅ est choisi parmi (CH₂)₆ ;
- R₆ est le 1,4:3,6-dianhydrosorbitol (DAS) selon la formule II ;
- R₇ est H, un groupe protecteur benzylique ou un agent ophtalmologique ; et
- R₈ est (CH₂)₄.
